Europäisches Patentamt

European Patent Office

Office européen des brevets

(19)

(11) Numéro de publication: **0 122 849**
**B1**

# (12) FASCICULE DE BREVET EUROPEEN

(45) Date de publication du fascicule du brevet:
**01.04.87**

(51) Int. Cl.⁴: **A 61 B 6/00,** F 16 M 11/10

(21) Numéro de dépôt: **84400683.3**

(22) Date de dépôt: **06.04.84**

(54) **Statif d'exploration isocentrique.**

(30) Priorité: **12.04.83 FR 8305940**

(43) Date de publication de la demande:
**24.10.84 Bulletin 84/43**

(45) Mention de la délivrance du brevet:
**01.04.87 Bulletin 87/14**

(84) Etats contractants désignés:
**DE NL**

(56) Documents cités:
**EP - A - 0 069 607**
**FR - A - 2 342 704**
**FR - A - 2 363 316**
**FR - A - 2 369 823**
**GB - A - 2 086 700**
**US - A - 3 617 749**

(73) Titulaire: **THOMSON-CGR, 13, square Max-Hymans,
F-75015 Paris (FR)**

(72) Inventeur: **Chambron, Edmond, THOMSON-CSF
SCPI 173, bld. Haussmann, F-75379 Paris Cedex 08 (FR)**

(74) Mandataire: **Grynwald, Albert et al, THOMSON-CSF
SCPI 19, avenue de Messine, F-75008 Paris (FR)**

ACTORUM AG

## Description

L'invention concerne un statif d'exploration isocentrique, destiné à permettre une exploration isocentrique d'un patient dans le cadre général du radiodiagnostic et, tout particulièrement dans celui de l'angiographie.

L'exploration isocentrique d'un patient consiste à faire en sorte que l'axe d'un faisceau de rayonnement X, où axe de travail, déterminé par l'alignement entre une source et un détecteur de rayonnement, passe toujours en un même point d'une zone à analyser, quelque soit l'incidence de cet axe; ce point constituant l'isocentre.

Il est connu à cette fin d'utiliser des statifs dans lesquels, un arceau de forme circulaire supporte la source et le détecteur de rayonnement; un déplacement circulaire dans son plan de l'arceau, provoquant le déplacement de la source et du détecteur ainsi qu'une rotation, autour de l'isocentre, de l'axe du faisceau qui reste dans un même plan.

De tels statifs peuvent être différenciés par le nombre de degrés de liberté qu'ils comportent, ou nombre d'axes selon lesquels l'arceau peut être mis en mouvement.

Un premier type de statif à plus de deux degrés de liberté, comporte généralement un arceau ouvert, aux extrémités duquel sont supportés la source et le détecteur de rayonnement. Une difficulté dans cette configuration, compte tenu des masses en mouvement, est qu'il exsite des porte-à-faux et des efforts de flexion importants. Aussi, un inconvénient de ce premier type de statif, est que le mouvement de l'arceau seul dans un même plan a une amplitude limitée; ce mouvement de l'arceau ne permettant pas par exemple de rétablir l'axe de travail initial, en remplaçant l'un par l'autre la source et le détecteur de rayonnement X, ni d'effectuer des incidences latérales avant ou arrière, sans nécessiter une manœuvre complexe où des mouvements sont effectués selon plus de deux axes. Un tel dispositif est connu de la demande de brevet FR-A-2 342 704.

On trouve également un second type de statif à un ou deux degrés de liberté, dans lesquels la source et le détecteur de rayonnement sont supportés par un arceau circulaire fermé; il est alors possible d'obtenir une rotation de l'arceau seul, dans un même plan, d'amplitude beaucoup plus importante que dans le premier cas.

Le principal défaut d'un statif à arceau fermé, est qu'il limite des deux côtés l'accès au patient et, qu'il ne peut effectuer une rotation du plan de l'arceau par rapport à un axe vertical passant par le centre de cet arceau, à cause du panneau support de patient.

D'autre part, de la demande de brevet Européen EP-A2-0 069 607 est connu une installation de radiologie comprenant un système radiographique principal composé de deux supports porte-charge à structure télescopique, l'un supportant la source, l'autre le récepteur. La rotation de l'axe de travail autour de l'isocentre est d'amplitude limitée et nécessite des moyens de synchronisation des movements.

La présente invention concerne un statif pour exploration isocentrique, assurant à un arceau ouvert des conditions de déplacements circulaires dans un même plan, qui permettent des incidences latérales ainsi que de reconstituer l'axe de travail initial, en remplaçant sur cet axe la source de rayonnement et le détecteur l'un par l'autre. Ces possibilités sont obtenues grâce à un agencement du statif selon l'invention qui lui permet d'offrir un encombrement faible et un accès aisé aux côtés du patient, et de présenter des porte-à-faux et des efforts de flexion considérablement réduits.

Selon l'invention, un statif d'exploration isocentrique, destiné à l'examen radiologique d'un patient, comportant:

– un arceau ouvert en forme d'arc de cercle, déplaçable circulairement dans le plan qui le contient autour d'un axe de rotation passant par son centre;

– des premiers moyens de déplacement;

– une source et un détecteur de rayonnement X, supportés par l'arceau de manière à réaliser un axe de travail parallèle au plan de l'arceau et interceptant l'axe de rotation en un point isocentre;

– est caractérisé en ce que l'arceau est supporté par un support mobile en forme d'arc de cercle par rapport auquel s'effectue son déplacement, grâce aux premiers moyens de déplacement, ce support mobile étant lui-même supporté par un socle et déplaçable circulairement par rapport à ce socle, grâce à de seconds moyens de déplacements, ces déplacements conjugés de l'arceau et du support mobile s'effectuant autour du même axe de rotation et dans des plans confondus ou parallèles, de manière à permettre une rotation de l'axe de travail d'un angle égal ou supérieur à 180° autour de l'isocentre.

L'invention sera mieux comprise grâce à la description qui suit, et aux trois figures annexées, parmis lesquelles:

– la figure 1 montre par une vue en perspective un statif selon l'invention;

– la figure 2 montre par une vue en coupe transversale, une disposition d'éléments constitutifs du statif selon l'invention;

– la figure 3 montre schématiquement les possibilités de positionnement d'un arceau et d'un support mobile dont est muni le statif selon l'invention.

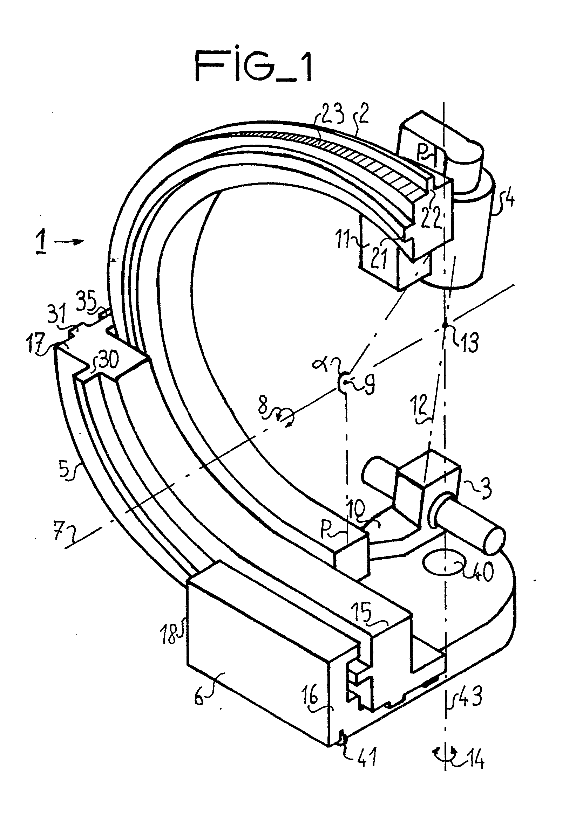

La figure 1 montre un statif 1 selon l'invention, permettant l'exploration isocentrique d'un patient disposé sur un panneau porte-patient, ces derniers n'étant pas représentés.

Le statif 1 comporte un arceau 2 ouvert, en forme d'arc de cercle, supportant une source de rayonnement X 3 et un détecteur de rayonnement X 4. L'arceau 2 est supporté par un support mobile 5 en forme d'arc de cercle également, ce support mobile 5 étant supporté à son tour par un socle 6.

L'arceau 2, dont l'arc de cercle qu'il constitue est inscrit dans un angle α égal ou supérieur à 180°, est destiné à accomplir un déplacement circulaire dans le plan qui le contient et autour d'un axe de rotation 7, comme montré par la flèche 8;

l'axe de rotation 7 passant par le centre 9 de l'arceau 2.

Dans l'exemple non limitatif décrit, la source 3 et le détecteur 4 de rayonnement X sont solidarisés à l'arceau 2, grâce à des moyens de fixation 10, 11, et occupent le long de cet arceau des positions P latérales; ces positions P permettent l'alignement de cette source 3 et de ce détecteur 4 selon un axe qui, en fonctionnement, constitue un axe de travail 12 interceptant l'axe de rotation 7. Le point d'intersection de l'axe de travail 12 et de l'axe de rotation 7 représente un point isocentre 13; l'axe de travail 12 passant toujours par cet isocentre 13 autour duquel il est mis en rotation, parallèlement au plan de l'arceau 2 comme montré par la flèche 8, du fait du déplacement circulaire de cet arceau 2.

Afin d'accroître cette rotation de l'axe de travail 12, le statif 1 selon l'invention permet également, en plus du déplacement circulaire de l'arceau 2, un déplacement circulaire du support mobile 5. Le déplacement du support mobile 5 s'opère parallèlement à celui de l'arceau 2, autour du même axe de rotation 7 que ce dernier, par rapport au socle 6. Dans l'exemple non limitatif décrit, la course du support mobile est comprise entre une position où sa première extrémité 15 coïncide avec l'avant 16 du socle 6, et une autre position où son autre extrémité 17 coïncide avec l'arrière 18 du socle 6.

Ainsi dans le statif 1 selon l'invention, d'une part l'arceau 2 seul est capable d'un déplacement circulaire autour de l'axe de rotation 7, entraînant autour de l'isocentre 13 la rotation de l'axe de travail 12, et d'autre part, le support mobile 5 entraînant l'arceau 2, peut lui-même être déplacé dans un mouvement circulaire autour de l'axe de rotation 7, par rapport au socle 6. Cette disposition permet à l'arceau 2 de coopèrer avec le support mobile 5, pour accroître la rotation de l'axe de travail 12 autour de l'axe de rotation 7 et particulièrement autour de l'isocentre 13, par les déplacements circulaires conjugués du support mobile 5 et de l'arceau 2.

Les déplacements de l'arceau 2 par rapport au support mobile 5, et du support mobile 5 par rapport au socle 6, s'effectuent respectivement grâce à des premiers et des seconds moyens de déplacement, partiellement représentés sur la figure 1.

Dans l'exemple non limitatif de la description, les premiers moyens de déplacement montrés par la figure 1 comportent notamment une première et une seconde nervure saillante 21, 22, solidaires de l'arceau 2 dont ils épousent la forme d'arc de cercle et qui coopèrent avec des premier et second moyens de roulement (non visibles sur la figure 1) dont est muni le support mobile 5, pour réaliser le guidage de l'arceau 2; ce dernier comporte en outre un premier moyen de transmission de mouvement 23, coopèrant avec des premiers moyens moteurs (non visibles sur la figure 1) que comporte le support mobile 5.

Les seconds moyens de déplacement montrés sur la figure 1 comportent également, dans l'exemple non limitatif décrit, une troisième et quatrième nervure saillante 30, 31, solidaires du support mobile 5 dont ils épousent la forme d'arc de cercle et qui coopèrent avec des troisième et quatrième moyens de roulement (non visibles sur la figure 1) solidaires du socle 6, pour réaliser le guidage du support mobile 5; des second moyens de transmission de mouvement 35, situés sur la périphérie du support mobile 5, coopèrent avec des seconds moyens moteurs (non représentés sur la figure 1), pour assurer le déplacement du support mobile 5 par rapport au socle 6.

Afin de permettre que la rotation de l'axe de travail 12 autour de l'isocentre 13 s'effectue dans d'autres plans, il est procédé à un déplacement circulaire du socle 6. A cet effet, le statif 1 selon l'invention comporte en outre de troisième moyen de déplacement. Ces troisièmes moyens de déplacement sont constitués dans l'exemple non limitatif de la description, par un pivot 40 et des cinquièmes moyens de roulement 41; le pivot 40 étant disposé selon un axe longitudinal confondu avec un axe vertical 43, passant par l'isocentre 13.

Ceci permet au socle 6 d'accomplir un déplacement circulaire dans lequel il entraîne le support mobile 5 et l'arceau 2, selon la flèche 14 autour de l'axe vertical 41, lequel constitue un second axe de rotation. Ce déplacement circulaire du socle 6 s'effectuant autour de l'axe vertical 43 passant par le point isocentre 13, cet isocentre 13 est conservé pour toutes les positions (non représentées) susceptibles d'être occupées par l'axe de travail 12, du fait des déplacements de l'arceau 2, du support mobile 5 et du socle 6.

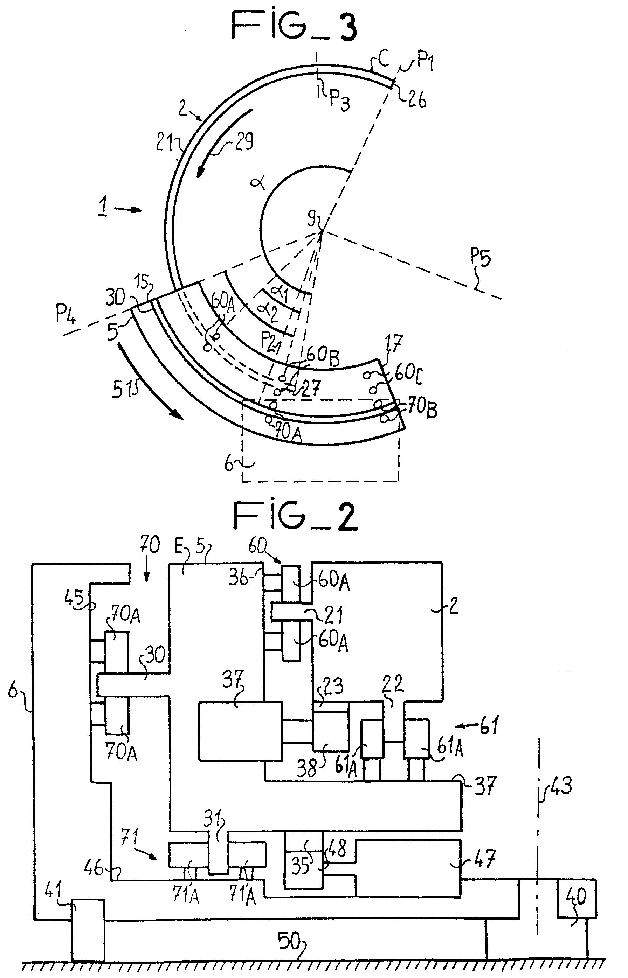

La figure 2 montre, par rapport à une coupe transversale de l'arceau 2, du support mobile 5 et du socle 6, une disposition des premiers, seconds et troisièmes moyens de déplacements.

Le guidage de l'arceau 2 est obtenu par une coopération entre les premières et secondes nervures saillantes 21, 22, et les premiers et seconds moyens de roulement précédemment mentionnés; dans l'exemple non limitatif décrit, ces moyens de roulement sont constitués par une première et seconde série 60, 61 de paires de paliers à rouleaux, dont une unique paire 60a, 61a est visible sur la figure 2. La première série 60 de paliers à rouleaux est fixée sur une face intérieure latérale 36 du support mobile 5, et roule sur des faces opposées de la première nervure saillante 21, réalisant un premier guidage de l'arceau 2 dans un plan vertical; la seconde série 61 de paliers à rouleaux est fixée sur une face intérieure inférieure 37 du support mobile 5 et roule en appui sur des faces opposées de la seconde nervure saillante 22, réalisant un second guidage de l'arceau 2, dans un plan transversal à celui de son premier guidage. Un premier moteur 37 disposé dans l'épaisseur E du support mobile 5, prolongé par une poulie crantée 38 en prise avec le moyen de transmission de mouvement 23 disposé le long de l'arceau 2, assure le déplacement de ce dernier; le moyen de transmission 23 étant dans l'exemple non limitatif décrit constitué par une crémaillère.

Les premières et secondes nervures saillantes 21, 22, les premiers et seconds moyens de roulement 60, 61, le premier moteur 37, la première poulie 38, et le premier moyen de transmission de mouvement 23, constituent les premiers moyens de déplacement 21–22, 60–61, 37, 38, 23.

Les troisièmes et quatrièmes nervures saillantes 30, 31 disposées le long du support mobile 5, coopèrent pour le guidage de ce dernier, avec les troisièmes et quatrièmes moyens de roulement précédemment mentionnés; dans l'exemple non limitatif décrit, ces troisièmes et quatrièmes moyens de roulement sont constitués par une troisième et une quatrième série 70, 71 de paires de paliers à rouleaux dont une unique paire 70a, 71a est visible sur la figure 2. La troisième série 70 de paliers à rouleaux est fixée sur une paroi intérieure 45 du socle 6, et roule sur des faces opposées de la troisième nervure saillante 30 que comporte le support mobile 5, réalisant un guidage de ce dernier dans un plan vertical; la quatrième série de paliers à rouleaux 71 est fixée sur une face de fond 46 du socle 6, et roule en appui sur des faces opposées de la quatrième nervure saillante 31 du socle mobile 5, réalisant un second guidage du support mobile 5 dans un plan transversal à celui de son premier guidage. Un second moteur 47, solidarisé à la face de fond 46 du socle 6, est prolongé par une seconde poulie crantée 48; cette seconde poulie est en prise avec le second moyen de transmission de mouvement 35 disposé le long du support mobile 5, parallèlement aux troisième et quatrième nervures saillantes, et assure grâce au second moteur 48 le déplacement du support mobile 5. Ce second moyen de transmission de mouvement 35 pouvant être constitué par exemple, comme dans le cas de l'arceau 2, par une crémaillère. Les troisième et quatrième séries 70, 71 de paires de paliers à rouleaux, les troisièmes et quatrièmes nervures saillantes 30, 31, le second moteur 47, le second moyen de transmission 35 et la seconde poulie crantée 48 constituent les seconds moyens de déplacements 70–71, 30–31, 47, 48, 35.

Le socle 6 repose sur le sol 50, par l'intermédiaire d'une part du pivot 40 dont l'axe longitudinal est confondu avec le second axe de rotation 43 vertical, et d'autre part par l'intermédiaire des cinquièmes moyens de roulement 41. Le déplacement circulaire du socle 6 autour du second axe de rotation 43, peut s'effectuer sous l'effet d'une pression manuelle ou, grâce à un moteur (non représenté) disposé pour entraîner d'une manière connue les moyens de roulement 41.

La figure 3 représente schématiquement l'arceau 2, le support mobile 5, et le socle 6 ainsi que la disposition, par rapport à ces derniers, des première et troisième séries 60, 70 de paires de paliers à rouleaux 60a, 60b, 60c, et 70a, 70b, 70c. Le socle 6 est symbolisé par un rectangle en traits pointillés, l'arceau 2 par la première nervure saillante 21, et le support mobile 5 est représenté avec la troisième nervure 30 afin d'éviter, pour une meilleure clarté du dessin, d'avoir à représenter les seconde et quatrième séries 61, 71 mentionnées précédemment; leur fonctionnement étant respectivement le même que les première et troisième séries 60, 70.

Dans la position occupée par l'arceau 2 sur la figure 3, celui-ci est maintenu sur le support mobile 5 par deux paires de paliers à rouleaux 60a, 60b, sur les trois paires 60a, 60b, 60c, qui dans l'exemple non limitatif décrit, constituent la première série 60.

Dans l'invention, la course de l'arceau 2 est limitée par la condition suivante:
– que son maintien soit toujours obtenu par au moins deux des trois paires de paliers à rouleaux 60a, 60b, 60c, selon qu'il est à l'une ou l'autre extrémité de sa course, soit par la paire central 60b et l'une ou l'autre des paires extrêmes 60a, 60c. Dans ces conditions le seul déplacement circulaire de l'arceau 2 selon la flèche 29, autour de son centre 9, permet à la première extrémité 26 de l'arceau d'aller d'une première position P1 à une seconde position P2. Dans la nouvelle position (non représentée) occupée par l'arceau 2, ce dernier est maintenu par deux paires de paliers 60b, 60c constituées par la paire centrale 60b et l'autre paire extrême 60c; sa seconde extrémité 27 étant alors à une position P3. Un point quelconque de l'arceau 2, comme le point C, par exemple, peut décrire un cercle correspond à un angle α dans lequel est inscrit l'arceau 2, moins un angle α 1 correspondant à la distance angulaire entre le palier central 60b et l'un des paliers extrêmes 60a, 60c. Une telle disposition permet à un arceau 2 muni d'une source 3 et d'un détecteur 4 de rayonnement X d'un type courant (non représentés sur la figure 3), d'accomplir une rotation de 180° avec des portes à faux réduits.

A la rotation seule de l'arceau 2, peut s'ajouter la rotation du support mobile 5. Ce dernier est lié au socle 6, ainsi qu'il a été mentionné précédemment, par une troisième et une quatrième série 70, 71 de chacune deux paires de paliers à rouleaux 70a, 70b et 71a, 71b: ces deux dernières paires n'étant pas représentées sur la figure 3.

Tel qu'il est représenté, le support mobile 5 est situé à l'une des positions extrêmes de sa course et peut effectuer un déplacement circulaire, ainsi que précédemment expliqué, autour de l'axe de rotation 7, qui sur la figure 3 est confondu avec le centre 9; ce déplacement circulaire du support mobile 5 peut s'effectuer dans des limites imposées par le fait qu'il doit être toujours maintenu par les deux paires de paliers à rouleaux 70a, 70b. Aussi le support mobile 5 peut effectuer un déplacement, dans le sens de la flèche 51, jusqu'à ce que l'une de ses extrémités 15, 17 arrive au niveau de l'un des paliers à rouleaux 70a, 70b. Ce déplacement peut avoir la valeur d'un angle α 2, correspondant dans l'exemple décrit au déplacement de la première extrémité 15, d'une position P4 jusqu'au niveau du palier à rouleaux 70a situé à la position P2; la seconde extrémité 17 étant alors à une nouvelle position P5.

Le support mobile 5 occupe alors une autre position extrême de sa course (non représentée), par rapport à laquelle l'arceau 2 peut effectuer son déplacement circulaire tel que précédemment mentionné.

Cette description de la répartition et du fonctionnement de la première série 60 de paires de paliers à rouleaux 60a, 60b, 60c est valable également pour la seconde série 61, de même que la description relative à la troisième serie 70 de paliers à rouleaux 70a, 70b est valable pour la quatrième série 71.

Ces possibilités de déplacement de l'arceau 2 et du support mobile 5, permettent d'obtenir une rotation de l'axe de travail 12 montrée sur la figure 1, dont l'amplitude correspond au déplacement de l'arceau 2 plus celui du support mobile 5. Il est à remarquer en outre que le statif 1 conforme à l'invention peut présenter un faible encombrement, en faisant coïncider les premières ou les secondes extrémités 26–15, 27–17 de l'arceau 2 et du support mobile 5.

Cette description constitue un exemple non limitatif, les structures de l'arceau 2 et du support mobile 5 pouvant être différente, et leur moyen de déplacement agencés différemment également. L'essentiel étant qu'un statif 1 d'exploration isocentrique comporte un arceau 2 en arc de cercle, mobile dans son plan autour de son centre 9 et par rapport à un support 5 en arc de cercle; ce support étant également mobile autour d'un même axe de rotation 7 que celui de l'arceau, et dans un plan confondu ou parallèle à celui de ce dernier.

## Revendications

1. Statif d'exploration isocentrique, destiné à l'examen radiologique d'un patient, comportant:

– un arceau (2) ouvert en forme d'arc de cercle, déplaçable circulairement dans le plan qui le contient autour d'un axe de rotation (7) passant par son centre (9);

– des premiers moyens de déplacements (21–22, 60–61, 37, 38, 23);

– une source (3) et un détecteur (4) de rayonnement X, supportés par l'arceau (2) de manière à réaliser un axe de travail (12) parallèle au plan de l'arceau (2) et interceptant l'axe de rotation (7) en un point isocentre (13);

– caractérisé en ce que l'arceau (2) est supporté par un support mobile (5) en forme d'arc de cercle par rapport auquel s'effectue son déplacement, grâce aux premiers moyens de déplacement (21–22, 60–61, 37, 38, 23), ce support mobile (5) étant lui-même supporté par un socle (6) et déplaçable circulairement par rapport à ce socle (6) grâce à de seconds moyens de déplacements (70–71, 30–31, 47, 48, 35), ces déplacements conjugués de l'arceau (2) et du support mobile (5) s'effectuant autour du même axe de rotation (7) et dans des plans confondus ou parallèles, de manière à permettre une rotation de l'axe de travail (12) d'un angle égal ou supérieur à 180° autour de l'isocentre (13).

2. Statif selon la revendication 1, caractérisé en ce qu'il comporte en outre de troisième moyens de déplacement (40, 41), permettant au socle (6) d'accomplir un déplacement circulaire autour d'un axe vertical (43) passant par l'isocentre (13).

3. Statif selon l'une des revendications précédentes, caractérisé en ce que les premiers moyens de déplacement (21–22, 60–61, 37, 38, 23) comportent une première et une seconde nervures saillantes (21, 22) coopérant respectivement avec des premiers et des seconds moyens de roulement (60, 61).

4. Statif selon la revendication 3, caractérisé en ce que les premiers et seconds moyens de roulement (60, 61) sont constitués respectivement par une première et une seconde série de paires de paliers à rouleaux (60a, 60b, 60c et 61a, 61b, 61c) dont au moins une paire centrale (60b, 61b) coopèrent en permanence avec une paire extrême (60a, 61a ou 60c, 61c) pour maintenir l'arceau (2) à l'une ou l'autre extrémité de sa course.

## Patentansprüche

1. Stativ zur isozentrischen radiologischen Untersuchung eines Patienten,

– mit einem offenen Bogen (2) in Form eines Kreisbogens, der kreisförmig in der ihn enthaltenden Ebene um eine durch sein Zentrum (9) verlaufende Rotationsachse (7) bewegt werden kann,

– mit ersten Verschiebemitteln (21–22, 60–61, 37, 38, 23),

– mit einer Röntgenstrahlenquelle (3) und einem Röntgenstrahlendetektor (4), die vom Bogen (2) so getragen werden, dass sie eine Arbeitsachse (12) bilden, die parallel zur Ebene des Bogens (2) liegt und die Rotationsachse (7) an einem isozentrischen Punkt (13) schneidet,

– dadurch gekennzeichnet, dass der Bogen (2) von einem beweglichen Träger (5) in Form eines Kreisbogens getragen wird, in bezug auf den er durch die ersten Verschiebemittel (21–22, 60–61, 37, 38, 23) bewegt wird, wobei dieser bewegliche Träger (5) seinerseits von einem Sockel (6) getragen wird und in bezug auf diesen Sockel (6) aufgrund zweiter Verschiebemittel (70–71, 30–31, 47, 48, 35) bewegt wird, wobei diese gemeinsamen Bewegungen des Bogens (2) und des beweglichen Trägers (5) um die gleiche Rotationsachse (7) herum und in einer gemeinsamen Ebene oder in parallelen Ebenen erfolgen, um eine Rotation der Arbeitsachse (12) um einen Winkel gleich oder grösser 180° um das Isozentrum (13) zu ermöglichen.

2. Stativ nach Anspruch 1, dadurch gekennzeichnet, dass es ausserdem dritte Verschiebemittel (40, 41) aufweist, die es dem Sockel (6) ermöglichen, eine kreisförmige Bewegung um eine durch das Isozentrum (13) verlaufende senkrechte Achse (43) durchzuführen.

3. Stativ nach einem der vorhergehenden Ansprüche, dadurch gekennzeichnet, dass die ersten Verschiebemittel (21–22, 60–61, 37, 38, 23) eine erste und eine zweite vorspringende Rippe (21, 22) aufweisen, die mit ersten bzw. zweiten Rollmitteln (60, 61) zusammenwirken.

4. Stativ nach Anspruch 3, dadurch gekennzeichnet, dass die ersten und zweiten Laufmittel (60, 61) aus einer ersten bzw. zweiten Reihe von Paaren von Rollenlagern (60a, 60b, 60c und 61a, 61b, 61c) bestehen, von denen mindestens ein zentrales Paar (60b, 61b) permanent mit einem

**0 122 849**

Aussenpaar (60a, 61a oder 60c, 61c) zusammenwirkt, um den Bogen (2) an dem einen oder anderen Ende seiner Laufbahn zu halten.

## Claims

1. A stand for isocentric exploration destined for the radiological examination of a patient, comprising:
– an arc-shaped open arch (2) which can be circularly displaced in the plane containing the same about a rotation axis (7) passing through its center (9),
– first displacement means (21–22, 60–61, 37, 38, 23),
– a X-ray source (3) and detector (4), supported by the arch (2) so as to obtain a working axis (12) parallel to the plane of the arch (2) and intercepting the rotation axis (7) at an isocentre point (13),
– characterized in that the arch (2) is supported by an arc-shaped moving support (5) relative to which its displacement takes place due to the first displacement means (21–22, 60–61, 37, 38, 23), said movable support (5) being itself supported by a base (6) and being circularly displaceable relative to said base (6) by second displacement means (70–71, 30–31, 47, 48, 35), these conjugated displacements of the arch (2) and the movable support (5) taking place about the same rotation axis (7) and in coinciding or parallel planes, so as to permit a rotation of the working axis (12) over an angle equal to or greater than 180° about the isocenter point (13).

2. A stand according to claim 1, characterized in that it further comprises third displacement means (40, 41) enabling the base (6) to perform a circular displacement about a vertical axis (43) passing through the isocenter (13).

3. A stand according to any one of the preceding claims, characterized in that the first displacement means (21–22, 60–61, 37, 38, 23) have first and second projecting ribs (21, 22) which respectively cooperate with first and second rolling means (60, 61).

4. A stand according to claim 3, characterized in that the first and second rolling means (60, 61) are respectively constituted by first and second groups of roller bearing pairs (60a, 60b, 60c and 61a, 61b, 61c) whereof at least one central pair (60b, 61b) permanently cooperates with an end pair (60a, 61a or 60c, 61c) to maintain the arch (2) at one or the other end of its travel.

FIG_1

# FIG_3

# FIG_2